# EUROPEAN PATENT SPECIFICATION

(11) **EP 2 948 160 B1**
(45) Date of publication and mention of the grant of the patent: **16.11.2016**
(21) Application number: 14704531.4
(22) Date of filing: 23.01.2014
(51) Int. Cl.: A61K 38/06, A61K 38/48, A61K 31/737, C08B 37/00, C12N 9/50, C12P 13/12, A61P 29/00

(54) **COMPOSITIONS CONTAINING CHONDROITIN SULPHATE, NATTOKINASE AND SULPHYDRYL COMPOUNDS FOR USE IN THE TREATMENT OF INFLAMMATION**
ZUSAMMENSETZUNGEN MIT CHONDROITINSULFAT, NATTOKINASE UND SULPHYDRYL-VERBINDUNGEN ZUR BEHANDLUNG VON ENTZÜNDUNGEN
COMPOSITIONS CONTENANT DU SULFATE DE CHONDROÏTINE, DE LA NATTOKINASE ET DES COMPOSÉS SULFHYDRYLÉS POUR LE TRAITEMENT DE L'INFLAMMATION

(30) Priority: 25.01.2013 IT MI20130117
(43) Date of publication of application: 02.12.2015
(73) Proprietor: Gnosis S.p.A., 20121 Milano (IT)
(72) Inventor: MIRAGLIA, Niccolò, I-20033 Desio (MI) (IT); ROSSINI, Mauro, I-20033 Desio (MI) (IT); BIANCHI, Davide, I-20033 Desio (MI) (IT); TRENTIN, Antonella, I-20033 Desio (MI) (IT)
(74) Representative: Bianchetti Bracco Minoja S.r.l.
(86) International application number: PCT/EP2014/051308
(87) International publication number: WO 2014/114706

(56) References cited:
- EP-A1- 1 304 338
- WO-A1-98/52583
- US-A1- 2004 241 256
- US-A1- 2009 110 674
- DATABASE WPI Week 201053 Thomson Scientific, London, GB; AN 2010-C20629 XP002697510, & RU 2 381 021 C2 (KLOPOTENKO L L) 10 February 2010 (2010-02-10)
- Seitaro Kamiya ET AL: "In vivo Evaluation Method of the Effect of Nattokinase on Carrageenan-Induced Tail Thrombosis in a Rat Model", Acta Haematologica (Basel), 13 November 2010 (2010-11-13), pages 218-224, XP055063676, DOI: 10.1159/000321518 Retrieved from the Internet: URL:http://www.karger.com/Article/Pdf/3215 18 [retrieved on 2013-05-22]

## Description

### SUMMARY OF THE INVENTION

The invention is defined by the claims. The present disclosure relates to compositions comprising chondroitin sulphate (CS), enzymes or enzymatic mixtures possessing proteolytic activity, either alone or in the presence of sulfhydryl compounds, and their use in the treatment and prevention of osteoarthritis and correlated acute and chronic inflammatory processes, or as nutraceutical compositions for the maintenance of musculoskeletal well-being in humans and animals.

Said combinations increase the intestinal absorption of CS when administered orally. The effect of said combinations is exerted on a wide range of molecular weights of CS, including samples consisting of oligosaccharides characterized by very low molecular weights (1-10 kDa), for which bioavailability greater than that of samples with a higher molecular weight is already known.

### TECHNICAL BACKGROUND

CS is recommended by EULAR (the European League against Rheumatism) as a symptomatic slow-acting drug for osteoarthritis (SYSADOA) in the treatment of osteoarthritis of the knee, hip and hand on the basis of extensive clinical evidence. CS is also used as a nutraceutical, either alone or combined with other ingredients, in compositions that perform an anti-inflammatory activity at both local and systemic levels.

Chondroitin sulphate (CS) is a polysaccharide belonging to the class of glycosaminoglycans (GAGs), which is present in both vertebrates and invertebrates and consists of disaccharide sequences formed by a residue of glucuronic acid (GlcA) alternating with a residue of N-acetyl-D-galactosamine (GalNAc), bonded together by beta 1-3 bonds and sulphated in different positions. The disaccharides are in turn bonded together by beta 1-4 bonds. CS mainly consists of monosulphated disaccharide units in the 4 position or the 6 position of GalNAc (called disaccharide A and C respectively). CS A and C are represented in different percentages, depending on the origin of the polysaccharide. Non-sulphated disaccharide and disulphated disaccharides bearing two sulphate groups bonded to the oxygen atom in various positions are also present in CS to a lesser, variable extent, depending on the specific animal sources: in the 2 position of GlcA and the 6 position of GalNAc (disaccharide D), in the 2 position of GlcA and the 4 position of GalNac, or in the 4 and 6 positions of GalNAc (disaccharide E) (Volpi N.J. Pharm. Pharmacol. 61, 1271, 2009. Volpi N.J. Pharm. Sci. 96, 3168, 2007. Volpi N. Curr. Pharm. Des. 12, 639, 2006).

CSs of different animal origins are also characterised by different molecular weights.

CSs originating from land animals, for example, have molecular mass values similar to one another but different from those originating from fish species, which have higher molecular mass values. CSs of land origin have a mean molecular weight ranging between 14 and 26 kDa, whereas CSs of marine origin, from squid, cartilaginous and bony fish, have a mean molecular weight exceeding 50 kDa.

In addition to CSs of animal origin, some CS products are described which are based on polysaccharide backbones of bacterial origin subsequently modified by synthesis to obtain polymers analogous to natural CS. These biotechnological CSs of bacterial-synthetic origin overcome some of the drawbacks associated with the animal origin of extracted CSs, such as the possible presence of viruses and/or prions or other potentially allergenic macromolecules among the residual impurities; the high animal protein content in the end product; the incompatibility of animal products with religious or dietary restrictions; and the limited sources available to meet growing worldwide demand.

Examples of CS of bacterial-synthetic origin are described, for example, in EP 1304338, wherein the capsular polysaccharide of *E. coli* strain O5:K4:H4 is chemically sulphated after extraction and hydrolysis of the original polymer. Other examples of bacterial-synthetic CS are disclosed in WO 2012/152872, WO 2012/159655 and WO 2013/174847, wherein a bacterial capsular polysaccharide is chemically sulphated to obtain CS similar to that of animal origin.

Finally, some examples of low-molecular-weight CS have been obtained by depolymerisation of extracted polysaccharides (Cho SY et al. Biol. Pharm. Bull. 27, 47, 2004, Das A. et al. Osteoart. Cartil. 8, 343, 2000), and polysaccharides of bacterial origin (WO 2013/174847, WO 2012/152872). The small molecular dimensions of these types of CS lead to better oral absorption, while maintaining many of the known activities of natural CS.

When administered orally, CS is absorbed by the intestinal mucosa in the small intestine and the distal tract. CS is partly absorbed as high-molecular-weight polysaccharide in the small intestine, while the majority is absorbed in the form of oligosaccharides in the caecum and colon (Lauder R.; Compl. Ther. Med. 17, 56-62, 2009). These oligosaccharides are generated by partial depolymerisation of the original polysaccharide by hydrolytic enzymes produced by the intestinal flora in the lower tract of the digestive apparatus.

Although the mechanism involved in the intestinal absorption of the polysaccharide constituting CS is not entirely clear, it is believed that absorption through the paracellular space of the intestinal epithelium is quantitatively significant, as is the case for most macromolecules; the oligosaccharide fragments of CS are also absorbed by this route. The tight junctions, which form a barrier that limits the absorption of large molecules whose transport does not involve mediation by specific molecular carriers through the intestinal mucosa, are present at this level.

The absorption of high-molecular-weight CS is estimated to amount to around 1-5%. As previously stated, the majority of CS absorbed consists of oligosaccharides deriving from its enzymatic digestion by chondroitinase produced by the intestinal microbial flora. Taking account of the absorption of oligosaccharides, however, the total uptake of CS by the intestinal mucosa amounts to no more than 20-23% of the polysaccharide ingested (Lauder R.; Compl. Ther. Med. 17, 56-62, 2009 - Barthe L. et al. Arzneimittelforsch./Drug Res. 2004; 54: 286-92).

In general, the absorption of CS after oral administration remains a problem, and any method able to increase the intestinal absorption of said glycosaminoglycan is highly topical.

Bromelain is a cysteine protease mixture extracted from the fruit and stem of the pineapple (*Ananas comosus*), a plant belonging to the Bromeliaceae family. The primary source for its extraction is the stem of the fruit, where its concentration is highest. Four separate fractions of this mixture can be distinguished or, according to a more elegant analytical characterisation conducted by mass spectrometry, eight proteolytic constituents, all with comparable proteolytic activity. The mixture in the natural form is normally used for this reason. Bromelain is classed as an endopeptidase belonging to the sub-family of C1A peptidases (MEROPS nomenclature). In addition to the protease mixture, the extract contains peroxidase, acid phosphatase and glycosidase. The molecular mass of the ingredients ranges between 8 and 28.5 kDa. The bromelain concentration is often expressed in proteolytic units (GDU units or international units, IU) rather than weight units.

In view of its proteolytic characteristics, bromelain has a similar activity to pancreatic protease, and therefore promotes digestion. It is used for dyspepsia in combination with pancreatic extracts. Bromelain also splits long-chain fats.

Bromelain also possesses other pharmacological activities, the most important of which are a potent anti-inflammatory activity that makes it effective in the treatment of inflammatory states of the soft tissues associated with traumas or post-operative reactions and local inflammations. It has been demonstrated that bromelain expresses this activity by increasing the biosynthesis of anti-inflammatory prostaglandins (such as E2 prostaglandins), and conversely by inhibiting the biosynthesis of pro-inflammatory prostaglandins.

Other pharmacological activities of bromelain include an anti-thrombotic and pro-fibrinolytic action, hypotensive activity and the ability to induce the regression of atherosclerotic plaque. Its synergic capacities in antibiotic and antitumoral treatments have also been described.

Bromelain is characterised by good oral bioavailability, estimated at around 40%, a rare characteristic for proteins. Moreover, the majority of unabsorbed bromelain remains intact, and able to express its enzymatic activity in the intestinal lumen, as it is not strongly attacked by the gastric juices or the cystatins present in saliva. In view of its characteristics it resembles papain and ficain, similar extracts deriving from papaya (*Carica papaya*) and figs (*Ficus carica*) respectively.

Nattokinase is an enzyme initially isolated from *natto,* a traditional Japanese food based on boiled soybeans fermented with a particular variety of *Bacillus subtilis, B. subtilis natto.*

Nattokinase is a serine protease of approx. 32 kDa which possesses potent fibrinolytic activity. Its homology with subtilisin exceeds 72%. Its fibrinolytic activities comprise a direct action in fibrinolysis together with the ability to induce an increase in the production of urokinase and plasmin. Nattokinase is characterised by relatively high stability to temperature and low pH values; this characteristic gives the protein good resistance to the gastric environment, allowing its use by oral administration.

Its characteristics of relative stability to the gastric environment and its proteolytic activity make it an enzyme which can present analogies with bromelain in the use illustrated here.

US 5679344 discloses nutrient compositions for use in joint disorders which contain glucosamine and proteolytic enzymes having anti-inflammatory properties. The compositions contain at least one protease and at least one acid-stabilised protease.

US 5888514 discloses compositions containing cartilage, proteolytic enzymes, glucosamine sulphate and chondroitin sulphate, together with vitamins and plant extracts, for use in the treatment of inflammation of the bones and joints.

### DESCRIPTION OF THE INVENTION

It has now been found that the combination of CS with bromelain and the simultaneous presence of a bromelain activity enhancer such as cysteine, methionine, glutathione or other sulfhydryl compounds increases its absorption in the small intestine.

It has also been found that nattokinase has an even more surprising effect on the bioavailability of CS, which is increased to over 250%.

The object of the present invention is a composition comprising chondroitin sulphate and one or more proteases, and optionally a sulfhydryl compound, provided that when the protease is other than nattokinase, the sulfhydryl compound is present.

"Sulphydryl compound" here means a natural or synthetic amino acid, or a small peptide or other compound comprising at least one sulphydryl group. The sulphydryl compound is preferably selected from methionine, cysteine, homocysteine, S-adenosylmethionine, acetylcysteine, reduced or oxidised glutathione and S-acetyl-glutathione.

In the compositions according to the invention, the chondroitin sulphate/protease/sulphydryl compound ratio is 1.0/0.05-0.8/0.001-0.05.

Chondroitin sulphate preferably has a molecular weight ranging between 1 and 95 kDa, more preferably between 4 and 50 kDa.

Chondroitin sulphate is preferably of extracted animal origin. CS can be obtained by chemical sulphation of capsular polysaccharide K4 from *E. coli* after removal of the fructose residues by hydrolysis, as described in EP 1304338, WO 2012/152872, WO 2012/159655, or by chemical sulphation and subsequent acid or radical depolymerisation of capsular polysaccharide K4 from *E. coli* after removal of the fructose residues by hydrolysis as described in WO 2013/174847 and WO 2012/152872. Alternatively, CS can be obtained by chemical sulphation of capsular polysaccharide from a genetically modified strain of *E. coli* (e.g. DSM23644), wherein the polysaccharide is originally devoid of fructose residues (WO 2012/159655). The molecular dimensions of the CS thus obtained can also be reduced subsequently by acid or radical depolymerization, as in WO 2013/174847.

The protease is preferably selected from bromelain, papain, ficain and nattokinase. Nattokinase is preferred. A preferred embodiment of the invention accordingly provides compositions comprising chondroitin sulphate and nattokinase, in the absence of sulphydryl compound. The invention also concerns the use of nattokinase for improving the intestinal permeability of chondroitin sulphate.

The compositions according to the invention can also contain one or more active ingredients used in the prevention or treatment of acute and chronic inflammatory states and/or one or more nutraceutical substances used to maintain musculoskeletal well-being in humans and animals.

The active ingredients can be selected, for example, from the group consisting of glucosamine hydrochloride, glucosamine sulphate, N-acetylglucosamine, hyaluronic acid, amino acids, collagen, hydrolysed collagen, polyunsaturated fatty acids, keratin, methylsulphonylmethane, folates, reduced folates, vitamins, Group B vitamins, S-adenosylmethionine (SAMe), ascorbic acid and manganese ascorbate.

The compositions can also contain one or more pharmaceutically or nutraceutically acceptable excipients.

All of the ingredients usually combined with chondroitin sulphate, such as glucosamine and methylsulphonylmethane (MSM), can also be added to said preparations.

Pharmaceutically or nutraceutically acceptable excipients are, for example, microcrystalline cellulose, stearic acid, magnesium stearate, colloidal silicon dioxide, ethylcellulose, methylcellulose, hydroxypropyl methylcellulose, aqueous shellac salts, sodium alginate, starch, modified starches, methacrylic acid copolymers, maltodextrins and polyols.

The compositions according to the invention are preferably administered orally, for example in the form of capsules, soft gel capsules, tablets, granulates, drinks in liquid form or powdered drinks to be reconstituted. The daily dose of CS can range between 400 mg and 3600 mg in the nutraceutical field, and the usual daily dose as a medicament is 1200 mg.

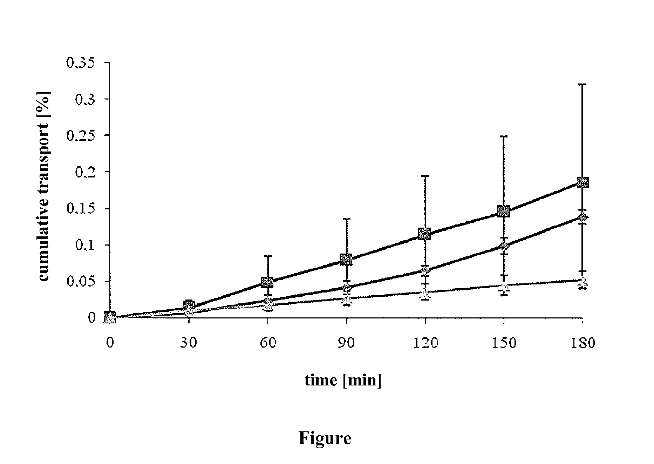

### DESCRIPTION OF THE FIGURE

The Figure shows the permeability through rat intestinal mucosa of bovine chondroitin sulphate, 20 kDa (diamonds), LMW chondroitin sulphate, 9 kDa (squares), and HMW chondroitin sulphate, 40 kDa (triangles).

### EXPERIMENTAL PART

The permeability of CS has been tested in an *in vitro* model wherein rat intestinal mucosa was excised from the animal immediately after euthanasia and placed in an Ussing chamber immersed in a suitable buffer at the interface of two compartments, with the side of the mucosa originally exposed to the intestinal lumen facing one compartment, called the donor compartment, and the basal part facing the other, called the acceptor compartment.

CS was placed in the donor compartment in the presence or absence of the other ingredients of the combination, and the presence of polysaccharides was determined in the acceptor compartment after an incubation period during which the CS permeated the acceptor compartment through the membrane consisting of rat intestinal mucosa.

The experimental technique used to evaluate the intestinal permeability of CS in its various combinations will now be described in more detail. Lewis rats weighing 150-170 g were euthanised by CO₂ inhalation and the small intestine was immediately excised, washed and mounted in an Ussing chamber filled with a medium consisting of 125 mM sodium chloride (NaCl), 1.3 mM magnesium sulphate (MgSO₄), 5 mM potassium chloride (KCl), 20 mM glucose and 25 mM sodium carbonate (NaHCO₃). The pH of the solution was adjusted to 7.4 with HEPES. The permeation studies were conducted at the temperature of 37°C in an atmosphere consisting of 95% O₂ and 5% CO₂. The permeation test was performed not more than 15 minutes after the excision of the intestinal mucosa.

The CS samples used in the test differed in terms of nature and molecular dimensions. CS samples of bovine origin and biotechnological (bacterial-synthetic) origin were subjected to the intestinal permeation test in the various combinations. The CS samples used were also characterised by different molecular weights, ranging between 1 and 95 kDa, or preferably between 4 and 50 kDa.

Chondroitin sulphate was added to the donor compartment at a concentration of 3% (mass/volume). For the permeation studies in the presence of bromelain, bromelain was added to the CS solution at the concentration of 1.5%. Alternatively, a sulphydryl compound selected from methionine, cysteine, homocysteine, S-adenosylmethionine, acetylcysteine, S-acetyl-glutathione and reduced or oxidised glutathione was added at the concentration of 0.075% together with bromelain. For the permeation studies in the presence of nattokinase, the enzyme was added to the solution at the concentration of 1.5%, as for the bromelain.

The total incubation period was three hours, during which 100 µl samples were taken every 30 min from the acceptor compartment, and the volume removed was replaced with fresh medium. The samples taken were analysed for the presence of the disaccharides making up CS by HPLC, using the method described below, after digestion of the polysaccharide with chondroitinase ABC (specific activity: 0.5 U/ml).

The HPLC method employed involved the use of a strong anion-exchange column (SAX), an eluent based on acidulated water at pH 4, and a linear gradient with 1.2 M NaCl from 0% to 100% in 25 min, after a first isocratic elution for 5 min in acidulated water only. The flow rate used was 1.0 ml/min, and detection of the disaccharides was effected at 232 nm with a UV detector.

The quantity of CS in the acceptor compartment was calculated with an eight-point calibration curve of standard chondroitin sulphate, corresponding to a range between 0.78% and 100% of the initial CS concentration C. The chondroitin sulphate standards had been pre-incubated with chondroitinase ABC, diluted in the same medium as used for the experiments. On the basis of the CS found in the acceptor compartment, the apparent permeability coefficient (Pₐₚₚ) was calculated with the formula *Pₐₚₚ (cm*/*sec) = Q*/*A·C·t,* wherein *Q* is the total quantity of CS permeated (µg), *A* is the diffusion area of the Ussing chamber (cm²), C is the initial concentration of CS in the donor compartment (µg/cm³), and *t* is the incubation time (30-180 min). The incremental ratio R was calculated on the Pₐₚₚ as *(Pₐₚₚ CS + protease*) / *(Pₐₚₚ CS* alone). Pₐₚₚ was calculated when each sample was taken, namely at 30, 60, 90, 120, 150 and 180 min. The average of the different Pₐₚₚ values obtained at these points was then calculated to obtain a mean permeability coefficient of every sample in the entire experiment. The CS permeation data were expressed as the average of the concentration peaks of disaccharides Δdi-0S, Δdi-6S and Δdi-4S, measured separately.

The statistical value of the data was analysed with Student's "t" test, with p < 0.05 as minimum significance.

The validity of this experimental model is confirmed by the fact that three samples of CS of different molecular weights, namely 9, 20 and 40 kDa, presented a permeability to the intestinal membrane which is a function of the molecular weight, as occurs *in vivo.* This is demonstrated by a graph showing the cumulative transport of CS at all time intervals considered within the 180-min period of the experiment (Figure).

Samples of CS of non-animal origin with a low (9 kDa) or high (40 kDa) molecular weight were then used in the permeability test in the presence or absence of the protease and, in the case of bromelain, with or without adding the enhancer compound.

The results of the permeation over time of the CS samples were obtained in the absence of other adjuvants or in the presence of bromelain, bromelain and methionine, or nattokinase.

Although the ability of bromelain to promote paracellular permeation of many macromolecules is known, this ability being associated with its ability to weaken the tight junctions (Grabovac et al., Int. J. Pharm. 326, 153-159, 2006), in this test, the use of bromelain alone, without the intervention of other factors, did not increase the absorption of low-molecular-weight CS. This can be seen from the comparison between the mean Pₐₚₚ values found, shown in table 1. However, an appreciable increase was observed for the combination of low-molecular-weight CS, bromelain and methionine. A surprising increase in the permeability of the intestinal mucosa was also observed in the case of the combination of low-molecular-weight CS and nattokinase (Table 1). The incremental ratios R have values exceeding 1 for the combination of CS/bromelain/methionine, and definitely higher than 1 for the combination of CS/nattokinase, whereas R is less than 1 for the combination of CS/bromelain (Table 2). Methionine therefore exhibits a surprisingly favourable effect on the action of bromelain as an enhancer of the intestinal permeability of CS, while even more surprisingly, nattokinase, without the intervention of other factors, nearly doubles the intestinal permeability of low-molecular-weight CS. It is interesting to note that the bioavailability of a low-molecular-weight CS, which is already more bioavailable than the corresponding large polysaccharide, can be further increased by the combinations described.

**Table 1**

| | **Pₐₚₚ (cm sec⁻¹) x 10⁻⁷** |
|---|---|
| LMW chondroitin sulphate | 2.13 ± 0.61 |
| LMW chondroitin sulphate + bromelain + methionine | 2.65 ± 1.36 |
| LMW chondroitin sulphate + bromelain | 1.41 ± 0.76 |
| LMW chondroitin sulphate + nattokinase | 3.56 ± 0.04 |

*Mean Papp values determined for the permeation of low-molecular-weight (LMW) chondroitin sulphate, either alone or combined with bromelain; bromelain* + *methionine; nattokinase*

**Table 2**

| | **Incremental Ratio (*R*)** |
|---|---|
| LMW chondroitin sulphate + bromelain + methionine/ LMW chondroitin sulphate | 1.25 ± 0.18 |
| LMW chondroitin sulphate + bromelain/ LMW chondroitin sulphate | 0.66 ± 0.35 |
| LMW chondroitin sulphate + nattokinase/ LMW chondroitin sulphate | 1.67 ± 0.20 |

### Incremental ratios of the combinations v. standalone low-molecular-weight (LMW) chondroitin sulphate

A comparable effect was observed for the absorption of high-molecular-weight CS (40 kDa), also of non-animal origin (Tables 3 and 4). In this case, the enhancing effect of the combinations is even more evident, almost triple the absorption of CS alone, indicating that the enhancing effect also takes place in the case of polysaccharides of large dimensions, for which absorption is more critical.

**Table 3**

| | **Pₐₚₚ (cm sec⁻¹) x 10⁻⁷** |
|---|---|
| HMW chondroitin sulphate | 0.53 ± 0.25 |
| HMW chondroitin sulphate + bromelain + methionine | 1.27 ± 0.80 |
| HMW chondroitin sulphate + nattokinase | 1.57 ± 1.10 |

*Mean Papp values determined for the permeation of high-molecular-weight (HMW) chondroitin sulphate, either alone or combined with bromelain; bromelain + methionine; nattokinase*

**Table 4**

| | **Incremental Ratio (*R*)** |
|---|---|
| HMW chondroitin sulphate + bromelain + methionine/HMW chondroitin sulphate | 2.42 ± 0.36 |
| HMW chondroitin sulphate + nattokinase/HMW chondroitin sulphate | 2.86 ± 1.64 |

### Incremental ratios of the combinations v. standalone high-molecular-weight (HMW) chondroitin sulphate

A sample of CS of animal origin (molecular weight: 15-20 kDa) was also subjected to the permeability test in the absence or presence of the cocktail of bromelain and methionine, confirming the ability of the combination to increase the permeability of CS from any source (Table 5).

**Table 5**

| | **Pₐₚₚ (cm sec⁻¹) x 10⁻⁷** |
|---|---|
| Reference chondroitin sulphate | 1.34 ± 1.24 |
| Reference chondroitin sulphate + bromelain + methionine | 1.45 ± 0.45 |

*Mean Papp values determined for the permeation of bovine chondroitin sulphate, either alone or combined with bromelain* + *methionine*

The following are examples of compositions according to the invention.

### Example 1

A composition was prepared by mixing:
1200 mg of bovine chondroitin sulphate,
600 mg of bromelain,
30 mg of L-methionine.

### Example 2

A composition was prepared by mixing:
1200 mg of bovine chondroitin sulphate,
600 mg of nattokinase.

### Example 3

A composition was prepared by mixing:
1200 mg of biotechnological chondroitin sulphate with a molecular weight of 9 kDa,
600 mg of bromelain,
30 mg of L-methionine.

### Example 4

A composition was prepared by mixing:
1200 mg of biotechnological chondroitin sulphate with a molecular weight of 9 kDa,
600 mg of nattokinase.

### Example 5

A composition was prepared by mixing:
1200 mg of biotechnological chondroitin sulphate with a molecular weight of 40 kDa,
600 mg of bromelain,
30 mg of L-methionine.

### Example 6

A composition was prepared by mixing:
1200 mg of biotechnological chondroitin sulphate with a molecular weight of 40 kDa,
600 mg of nattokinase.

### Example 7

A composition was prepared by mixing:
1200 mg of biotechnological chondroitin sulphate with a molecular weight of 9 kDa,
600 mg of nattokinase,
30 mg of L-methionine.

### Example 8

A composition was prepared by mixing:
1200 mg of biotechnological chondroitin sulphate with a molecular weight of 40 kDa,
600 mg of nattokinase,
30 mg of L-methionine.

### Example 9

A composition was prepared by mixing:
1200 mg of bovine chondroitin sulphate,
600 mg of nattokinase,
30 mg of L-methionine.

## Claims

1. A composition comprising chondroitin sulphate, nattokinase and optionally a sulphydrylated compound, the chondroitin sulphate/protease/sulphydrylated compound ratio being 1.0/0.05-0.8/0.001-0.05 by weight.

2. A composition according to claim 1, in the absence of a sulphydrylated compound.

3. A composition according to claim 1, in the presence of a sulphydrylated compound.

4. The composition according to any one of claims 1-3 wherein chondroitin sulphate has a molecular weight ranging from 1 to 95 kDa.

5. The composition according to any one of claims 1-4 wherein chondroitin sulphate is obtained by extraction from an animal source.

6. The composition according to any one of claims 1 and 4 wherein chondroitin sulphate is obtained by chemical sulphation of the capsular polysaccharide K4 of *E. coli* after removal of the fructose residues by hydrolysis.

7. The composition according to any one of claims 1 and 4 wherein chondroitin sulphate is obtained by chemical sulphation and subsequent acid or radical depolymerisation of the capsular polysaccharide K4 of *E*. *coli* after removal of the fructose residues by hydrolysis.

8. The composition according to any one of claims 1 and 4 wherein chondroitin sulphate is obtained by chemical sulphation of the capsular polysaccharide of a genetically modified strain of *E. coli,* in which said polysaccharide is originally free from fructose residues.

9. The composition according to any one of claims 1 and 4 wherein chondroitin sulphate is obtained by chemical sulphation and subsequent acid or radical depolymerization of the capsular polysaccharide of a genetically modified strain of *E. coli,* in which said polysaccharide is originally free from fructose residues.

10. The composition according to any one of claims 1-9 wherein sulphydrylated compound is selected from methionine, cysteine, homocysteine, S-adenosylmethionine, acetylcysteine, reduced or oxidized glutathione, S-acetyl-glutathione.

11. The composition according to any one of claims 1-10 further containing one or more active principles used in the prevention or treatment of acute or chronic inflammation and/or one or more nutraceutical substances used for maintaining the musculo-skeletal wellness in men and animals.

12. The composition according to claim 11 wherein the one or more active principles are selected from the group consisting of glucosamine hydrochloride, glucosamine sulphate, N-acetyl-glucosamine, hyaluronic acid, amino acids, collagen, hydrolysed collagen, polyunsaturated fatty acids, keratin, methylsulphonylmethane, folate, reduced folate, vitamins, group B vitamins, S-adenosylmethionine (SAMe), ascorbic acid and manganese ascorbate.

13. The composition according to any one of claims 1-12 further containing one or more excipients pharmaceutically o nutraceutically acceptable selected from microcrystalline cellulose, stearic acid, magnesium stearate, colloidal silica, ethylcellulose, methylcellulose, hydroxypropyl methylcellulose, shellac aqueous salts, sodium alginate, starch, modified starches, methacrylic acid copolymers, maltodextrins, polyols.

14. The composition according to any one of the claims 1-13 for the use in prevention or treatment of acute and chronic inflammations.

15. The composition according to any one of the preceding claims in solid oral formulations comprising capsules, soft gel capsules, tablets, granulates, liquid beverages or reconstituted powdered beverages.

## Patentansprüche

1. Eine Zusammensetzung, umfassend Chondroitinsulfat, Nattokinase und wahlweise eine sulfhydrylierte Verbindung, wobei das Verhältnis von Chondroitinsulfat/Protease/sulfhydrylierter Verbindung 1,0/0,05-0,8/0,001-0,05 nach Gewicht beträgt.

2. Eine Zusammensetzung gemäß Anspruch 1, in der Abwesenheit einer sulfhydrylierten Verbindung.

3. Eine Zusammensetzung gemäß Anspruch 1, in der Anwesenheit einer sulfhydrylierten Verbindung.

4. Die Zusammensetzung gemäß einem der Ansprüche 1-3, wobei Chondroitinsulfat ein Molekulargewicht im Bereich von 1 bis 95 kDa hat.

5. Die Zusammensetzung gemäß einem der Ansprüche 1-4, wobei Chondroitinsulfat durch Extraktion aus einer tierischen Quelle erhalten wird.

6. Die Zusammensetzung gemäß einem der Ansprüche 1 und 4, wobei Chondroitinsulfat durch chemische Sulfatierung des Kapselpolysaccharids K4 von *E. coli* nach Entfernung der Fruktosereste durch Hydrolyse erhalten wird.

7. Die Zusammensetzung gemäß einem der Ansprüche 1 und 4, wobei Chondroitinsulfat durch chemische Sulfatierung und anschließende Säure- oder Radikal-Depolymerisation des Kapselpolysaccharids K4 von *E. coli* nach Entfernung der Fruktosereste durch Hydrolyse erhalten wird.

8. Die Zusammensetzung gemäß einem der Ansprüche 1 und 4, wobei Chondroitinsulfat durch chemische Sulfatierung des Kapselpolysaccharids eines genetisch modifizierten *E. coli*-Stamms, in welchem besagtes Polysaccharid ursprünglich frei von Fruktoseresten ist, erhalten wird.

9. Die Zusammensetzung gemäß einem der Ansprüche 1 und 4, wobei Chondroitinsulfat durch chemische Sulfatierung und anschließende Säure- oder Radikal-Depolymerisation des Kapselpolysaccharids eines genetisch modifizierten *E. coli*-Stamms, in welchem besagtes Polysaccharid ursprünglich frei von Fruktoseresten ist, erhalten wird.

10. Die Zusammensetzung gemäß einem der Ansprüche 1-9, wobei die sulfhydrylierte Verbindung aus Methionin, Cystein, Homocystein, S-Adenosylmethionin, Acetylcystein, reduziertem oder oxidiertem Glutathion, S-Acetylglutathion ausgewählt ist.

11. Die Zusammensetzung gemäß einem der Ansprüche 1-10, ferner enthaltend einen oder mehrere aktive Wirkstoffe, welche bei der Prävention oder Behandlung akuter oder chronischer Entzündung verwendet werden, und/oder eine oder mehrere nutrazeutische Substanzen, welche zur Erhaltung des muskuloskelettalen Wohlbefindens in Menschen und Tieren verwendet werden.

12. Die Zusammensetzung gemäß Anspruch 11, wobei der eine oder die mehreren aktiven Wirkstoffe aus der Gruppe bestehend aus Glucosaminhydrochlorid, Glucosaminsulfat, N-Acetylglucosamin, Hyaluronsäure, Aminosäuren, Kollagen, hydrolysiertem Kollagen, mehrfach ungesättigten Fettsäuren, Keratin, Methylsulfonylmethan, Folat, reduziertem Folat, Vitaminen, Vitaminen der Gruppe B, S-Adenosylmethionin (SAM), Ascorbinsäure und Manganascorbat ausgewählt sind.

13. Die Zusammensetzung gemäß einem der Ansprüche 1-12, ferner enthaltend einen oder mehrere pharmazeutisch oder nutrazeutisch verträgliche Hilfsstoffe, ausgewählt aus mikrokristalliner Zellulose, Stearinsäure, Magnesiumstearat, kolloidalem Siliciumdioxid, Ethylzellulose, Methylzellulose, Hydroxypropylmethylzellulose, wässrigen Schellack-Salzen, Natriumalginat, Stärke, modifizierten Stärken, Methacrylsäure-Copolymeren, Maltodextrinen, Polyolen.

14. Die Zusammensetzung gemäß einem der Ansprüche 1-13 zur Verwendung bei Prävention oder Behandlung akuter und chronischer Entzündungen.

15. Die Zusammensetzung gemäß einem der vorhergehenden Ansprüche in festen Oralformulierungen, umfassend Kapseln, Weichgelkapseln, Tabletten, Granulate, Flüssiggetränke oder rekonstituierte Pulvergetränke.

## Revendications

1. Composition comprenant du sulfate de chondroïtine, de la nattokinase et éventuellement un composé sulfhydrylé, le rapport de sulfate de chondroïtine/protéase/ composé sulfhydrylé étant de 1,0/0,05 à 0,8/0,001 à 0,05 en poids.

2. Composition selon la revendication 1, en l'absence de composé sulfhydrylé.

3. Composition selon la revendication 1, en présence d'un composé sulfhydrylé.

4. Composition selon l'une quelconque des revendications 1 à 3, dans laquelle le sulfate de chondroïtine a un poids moléculaire allant de 1 à 95 kDa.

5. Composition selon l'une quelconque des revendications 1 à 4, dans laquelle le sulfate de chondroïtine est obtenu par extraction à partir d'une source animale.

6. Composition selon l'une quelconque des revendications 1 et 4, dans laquelle le sulfate de chondroïtine est obtenu par sulfatation chimique du polysaccharide capsulaire K4 de *E*. *coli* après élimination des résidus fructose par hydrolyse.

7. Composition selon l'une quelconque des revendications 1 et 4, dans laquelle le sulfate de chondroïtine est obtenu par sulfatation chimique et dépolymérisation acide ou radicalaire ultérieure du polysaccharide capsulaire K4 de *E*. *coli* après élimination des résidus fructose par hydrolyse.

8. Composition selon l'une quelconque des revendications 1 et 4, dans laquelle le sulfate de chondroïtine est obtenu par sulfatation chimique du polysaccharide capsulaire d'une souche génétiquement modifiée de *E. coli,* dans laquelle ledit polysaccharide est initialement exempt de résidus fructose.

9. Composition selon l'une quelconque des revendications 1 et 4, dans laquelle le sulfate de chondroïtine est obtenu par sulfatation chimique et dépolymérisation acide ou radicalaire ultérieure du polysaccharide capsulaire d'une souche génétiquement modifiée de *E. coli,* dans laquelle ledit polysaccharide est initialement exempt de résidus fructose.

10. Composition selon l'une quelconque des revendications 1 à 9, dans laquelle le composé sulfhydrylé est choisi parmi la méthionine, la cystéine, l'homocystéine, la S-adénosylméthionine, l'acétylcystéine, le glutathion oxydé ou réduit, le S-acétyl-glutathion.

11. Composition selon l'une quelconque des revendications 1 à 10, contenant en outre un ou plusieurs principes actifs utilisés dans la prévention ou le traitement d'une inflammation aiguë ou chronique et/ou une ou plusieurs substances nutraceutiques utilisées pour maintenir le bien-être musculo-squelettique chez l'homme et l'animal.

12. Composition selon la revendication 11, dans laquelle le ou les principes actifs sont choisis dans le groupe constitué par le chlorhydrate de glucosamine, le sulfate de glucosamine, la N-acétyl-glucosamine, l'acide hyaluronique, les acides aminés, le collagène, le collagène hydrolysé, les acides gras poly-insaturés, la kératine, le méthylsulfonylméthane, le folate, le folate réduit, les vitamines, les vitamines du groupe B, la S-adénosylméthionine (SAMe), l'acide ascorbique et l'ascorbate de manganèse.

13. Composition selon l'une quelconque des revendications 1 à 12, contenant en outre un ou plusieurs excipients pharmaceutiquement ou nutraceutiquement acceptables choisis parmi la cellulose microcristalline, l'acide stéarique, le stéarate de magnésium, la silice colloïdale, l'éthylcellulose, la méthylcellulose, l'hydroxypropylméthylcellulose, les sels de laque aqueux, l'alginate de sodium, l'amidon, les amidons modifiés, les copolymères d'acide méthacrylique, les maltodextrines, les polyols.

14. Composition selon l'une quelconque des revendications 1 à 13 pour l'utilisation dans la prévention ou le traitement des inflammations aiguës et chroniques.

15. Composition selon l'une quelconque des revendications précédentes, dans des formulations solides orales comprenant des gélules, des capsules molles, des comprimés, des granulés, des boissons liquides ou des boissons en poudre reconstituées.
